Europäisches Patentamt

European Patent Office

Office européen des brevets

(11) Publication number: **0 061 556**
**B1**

(12) **EUROPEAN PATENT SPECIFICATION**

(45) Date of publication of patent specification: **03.10.84**

(51) Int. Cl.³: **C 07 G 7/04** // A61K37/18

(21) Application number: **81850050.6**

(22) Date of filing: **20.03.81**

---

(54) Hemiron-enriched amino acid preparation and a process for the preparation of hemiron-enriched amino acid preparations from hemproteins.

---

(43) Date of publication of application:
**06.10.82 Bulletin 82/40**

(45) Publication of the grant of the patent:
**03.10.84 Bulletin 84/40**

(84) Designated Contracting States:
**AT BE CH DE FR GB IT LI LU NL**

(56) References cited:
**FR-A-2 399 213**
**GB-A- 673 063**
**US-A-4 167 564**

**CHEMICAL ABSTRACTS, vol. 87, no. 7, 15th August 1977, page 350, no. 51782k, Columbus, Ohio, U.S.A., K.J. STACHOWICZ et al.:**
**"Enzymic hydrolysis of ox-blood hemoglobin"**

(73) Proprietor: **AB Pripps Bryggerier**
**Voltavägen 29**
**S-161 86 Bromma (SE)**

(72) Inventor: **Eriksson, Caj**
**Näverlursvägen 1**
**S-435 00 Mölnlycke (SE)**

(74) Representative: **Atterby, Fred et al**
**Dr. Ludwig Brann Patentbyra AB Kungsgatan 3**
**Box 7524**
**S-103 92 Stockholm (SE)**

The file contains technical information submitted after the application was filed and not included in this specification

Courier Press, Leamington Spa, England.

## Description

The present invention relates to a hemiron-enriched amino acid preparation and a process for the preparation of hemiron-enriched amino acid preparations from hem-proteins, especially hemoglobin.

Hemoglobin in animal as well as human blood and also other natural hemoproteins consists of a great number of amino acids which are chemically bonded to each other in long chains. Certain hemproteins, e.g. peroxidase in plants, also contain carbohydrates. Hemoglobin contains four amino acid chains. A special group, a hemgroup, is connected to each chain. This hemgroup consists of porphyrin to which a central iron atom is bonded. Thus, each hemoglobin molecule consists of totally four amino acid chains with totally four hemgroups containing together four iron atoms. This hem gives the blood its oxygen-transporting ability. The hem also contributes to the colour of blood. Hem is bonded at the amino acid chain by a bonding between on the one side the iron atom of the hem and on the other to a special amino acid in the chain. There are several histidin groups in each chain but only one of them is thus used for the hem-bonding.

It is known that the iron which is administrated to the body in the form of hem, essentially from hemoglobin and myoglobin, in certain cases is better utilized than the other forms of iron, e.g. ferrum reductum or different iron salts which are the usual forms of iron for enrichment of foodstuffs, e.g. flour, drinks or for use in pharmaceutical iron preparations. The need for iron enrichment, e.g. in certain foodstuffs such as bread, is well documented.

The need for an iron-containing product with improved properties as regards uptake in the gastrointestinal tracts has been recognized previously and attempts have been made to prepare such products. GB—A—673 063 relates to metallic compounds of amino acids and their preparation. The product prepared consists of a complex admixture of amino acid salts of iron in a form stated to be substantially the same as that occurring in natural hemoglobin. The process for the preparation of the product comprises enzymatical digestion of a proteinous material to liberate the amino acids and reacting the same with selected iron salts.

An early attempt to hydrolyse ox-blood enzymatically under slightly alkaline conditions for obtaining protein hydrolysates with a very low heme content was described in C.A., 87, No. 7. August 15, 1977, page 350, No. 51782K.

US—A—4 167 564 relates to a method for improving the stability of amino acid-metal complexes or chelates and enhancing the uptake of these complexes into biological tissues. The method comprises forming the metal complexes with amino acids or hydrolyzed proteins and incorporating into them a buffer system which controls the pH of the complex and the surrounding media.

The present invention relates to hemeiron-enriched amino acid preparations and a process for their preparation which are of improved value for iron-enrichment of foodstuffs and beverages.

The hem iron is absorbed in the human body by another mechanism than other types of iron; there is a distinction between the absorption via the so-called hemiron pool and the non-hemiron pool. This partly explains why the utilization of hemiron in the body is influenced to a lesser extent by other foodstuff components than other forms of iron. Thus, the hemiron utilization is not influenced in a negative direction by for instance phytinic acid and its salts which are present in cereals and cereal products, or tannic acid in for instance tea, phosphate or other components in eggs, which is the case for other iron forms.

Other forms can, especially in high doses, give different side-reactions in man. The most important advantages with hemiron in diet contexts would thus be its inherent natural properties including independence of other foodstuff components in the utilization in the body and lesser side effects. An important condition for correct resorption of hem is, however, that it is bonded to a suitable carrier. Iron in hem is thus very poorly utilized if the hem first is separated from protein and administrated separately. This leads to the conclusion that the amino acid chain to which the hem originally is bonded should have a positive influence in this respect.

The hemoglobin molecule consists, calculated on the weight, predominantly of amino acids. In 1000 mg of hemoglobin the amino acids are together about 960 mg and hem only 40 mg, which is equivalent with 3.5 mg of iron, viz. only 4% by weight hem and 0.35% by weight of iron resp.

This means that a great amount of amino acids automatically must be administrated to the body in relation to the amount of iron if hemoglobin is utilized as a source of iron. If for instance the daily need of iron (15 mg) would be administrated in the form of hemoglobin simultaneously 4.2 g of amino acids (7% of the daily need of protein) would have to be consumed. A therapeutical dosis of 100 mg of iron daily would mean an extra addition of about 28 g of amino acids, which corresponds to nearly half of the daily need of protein. It is unrealistic to administrate such great amounts of amino acids above the amount of protein which is administrated through the food.

If hemoglobin would be used for iron-enrichment of foodstuffs all the protein simultaneously administrated would correspondingly harmfully influence the technical properties of the foodstuff and possibly also its nutritional value. Accordingly, there is a need for a hemiron product with the following properties:

2

O 061 556

— a higher hem content (and accordingly a higher iron content) than hemoglobin
— the hem is bonded in a similar manner as in hemoglobin
— good solubility properties for admixture in all types of foodstuffs and pharmaceutical preparations.

It has now been found that these requirements are fulfilled by a hemiron-enriched amino acid preparation prepared from hem proteins. The preparation is characterized in that its iron content exclusively consists of the porphyrin iron of the original hem protein bonded in this form (hem) to amino acid chains, which are considerably shorter than the amino acid chains in the original hem protein, viz. these chains have for achievement of the enrichment been made shorter. However, these amino acid chains have dimensions which are longer than the separate short, hemiron-containing amino acid chains which for instance has been achieved by aggregation. This will permit separation from amino acid chains which do not contain hemiron. It is suitable that the preparation has a constant ratio between hem and iron and a porphyrin hemiron content of at least 0.5% and preferably at the most 5%, calculated on the iron bonded to porphyrin.

The invention relates primarily to a process for the preparation of hemiron-enriched amino acid preparations from hemproteins, especially hemoglobin. The process is characterized in that the hemprotein in a manner known per se first is hemolyzed and denaturated and subsequently split with the aid of proteolytic enzymes to one hemiron-enriched and one hemiron-poor fraction and in that the hemiron-enriched fraction is obtained by separation, the hemiron-enriched fraction preferably being aggregated. The preparation is subsequently stabilized by heat-treatment and concentrated, preferably by drying.

The stabilization of the hemiron-enriched fraction is made by a heat-treatment in which the enzymes are inactivated. The most common treatment is pasteurization at for instance 70°C for 30 minutes and/or sterilization at 121°C for 5 minutes. Pasteurization is preferably made at 60—90°C for 30—10 minutes but can also be made at higher temperatures, e.g. 90—110°C for a shorter period of time. Sterilization is preferably made at 110—121°C for 20—5 minutes and can also be made at a higher temperature and for a correspondingly shorter period of time. The determination of a suitable temperature and a corresponding period of time is made by routine experiments.

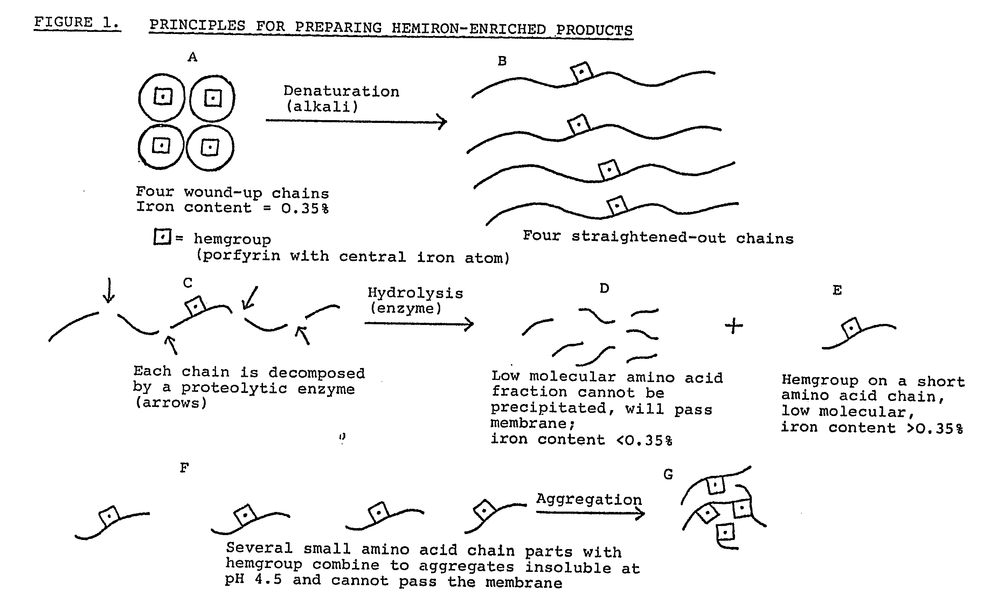

The invention is further elucidated with reference to the enclosed drawing on which Fig. 1A diagramatically shows a hemprotein molecule. Fig. 1B shows this molecule after denaturation. Fig. 1C shows one of the four straightened chains of denaturated protein according to Fig. 1B. Fig. 1D represents the amino acid fraction obtained after the reaction and Fig. 1E shows a hemiron-enriched amino acid preparation according to the invention. Finally, Fig. 1F shows how several such hemiron-enriched amino acid preparations are combined to aggregates.

As has been mentioned above a hemoglobin molecule consists of four amino acid chains, each of which contains a hem-group the iron atom of which is bonded to one of the histidin groups of the chain. These chains are in the natural condition winded up in a complex manner so that each hemgroup is located within the winded chain. The major part of the bondings which hold the chains together and also the hemgroup to the chain, are inaccessible for larger molecules, e.g. enzymes which can split the bondings which keep the chain together; however, the bondings are accessible for small molecules, e.g. oxygen, which is transported within the body bonded to hem. This natural condition is shown in Fig. 1 sub A. The winded-up chains can be "unrolled" to straighter chains with the aid of denaturating agents, e.g. urea, alkali, oxygen, heat, organic solvents or combinations thereof. The four chains in a hemoglobin molecule will then be separated from each other which is shown in Fig. 1 sub B. Each such "unrolled" chain is now accessible for the attack of a proteolytic enzyme which can break down the bonding between amino acids in an amino acid chain, e.g. in hemoglobin. Different proteolytic enzyme can break different such bondings but the result will always be that the length of the amino acid is shortened; this is shown in Fig. 1, C—E. Proteolytical enzymes can be obtained from organs from plants and animals and from microorganisms.

These proteolytic enzymes have the ability to break down the bondings between amino acids in an amino acid chain, e.g. hemoglobin. Different proteolytic enzymes break different such bondings but the result will in all cases be that the length of the amino acid chain is shortened; this is elucidated in Fig. 1C—E. The degree of decomposition is then determined by the nature of the enzyme, the time, the pH and the temperature at the interaction of the enzyme and the composition of the reaction products formed.

According to the present invention hemprotein is thus hydrolyzed with the aid of proteolytic enzymes so that superfluous amino acids are removed, whereas the natural hemiron is retained at the amino acid to which it was originally bonded, as many amino acids in the neighbourhood of this bonding location being retained so that the desirable technical and physiological properties are obtained or maintained resp. in the product. After the hydrolysis the hemprotein can be separated into two main fractions, viz. one containing the major part of the hemiron of the hemprotein having a hemiron content which is up to 16 times greater than the hemiron content of the original hemprotein, and a second fraction containing the major part of the amino acids of the hemprotein. The hemiron-rich fraction can during the reaction be given greater molecular dimensions than the second fraction which can be used for separation of the fractions.

3

It is important that the iron is concentrated in the hemiron-rich fraction without any addition of external iron, since the iron in the hemprotein and also in the hemiron-rich fraction has a special natural form with an especially good resorbability in the human body. The fraction obtained is, due to its high content of this specific iron, a good iron source for use in foodstuffs and pharmaceuticals (therapeutical use).

There are a number of proteins which consist of amino acids and hem, in the present context called hemproteins, among which hemoglobin, myoglobin, cytochrom, catalas and peroxidas are to be noted and occurring in the most cells of animal, vegetable and microbiological origin. The invention is applicable on all these hemproteins but hemoglobin is the only material which seems to be of a practical importance.

The mixture (hydrolysate) resulting from the decomposition of a hemprotein according to the invention has other properties than the original protein. The water-solubility of the hydrolysate is normally independent of the pH, whereas the water-solubility of the starting protein commonly is dependent on the pH of the solution. The enzyme does not act on the hemgroup per se in the process according to the invention.

The process defined above has hitherto not caused any greater difference as regards the molecular size between the hemiron-containing and the non-hemiron-containing fraction (1D—E). In an aggregation process the hemiron-containing fraction can, however, be given such molecular dimensions that it can easily be separated from the fraction not containing hemiron. This is shown in Fig. 1F—G. Such an aggregation can be achieved either by adjustment of the pH or by concentration. If the pH is adjusted to 4.5 the hemiron product will form aggregates so that it is precipitated and can be separated easily. If the concentration is increased by membrane filtration with the use of a tight membrane the hemiron fraction aggregates continuously and does not pass through the membrane. If the pH is adjusted to about 7 before ultrafiltration and without a previous precipitation a more porous membrane can be used.

As has been shown above it has, according to the invention, been achieved that the protein rests to which the hemgroup is bonded are given such a size that they can be used for separation of the hem-rich fraction (the hem-containing product). That such a size is achieved in the hem-rich protein rests seems to depend on the fact that either;

a) the hemiron-containing rests primarily have small dimensions but subsequently combine to the formation of greater aggregates due to hydrophobic interactions. These aggregates will then contain almost all of the hemiron and can be separated according to Fig. 2; or

b) part of the protein is left essentially unchanged during the enzymatic hydrolysis. These protein chains "take care of" the hemgroup which during the proceeding hydrolysis of other protein chains no longer can be bonded to the lesser aggregates. Then a superhemprotein would be formed, viz. a hemprotein with more than one hemgroup per amino acid chain, due to hydrophobic interaction.

The following experiment has been made for the purpose to decide which of the mechanisms a) and b) is probable:

250 mg of untreated hemoglobin and a hemiron product prepared according to A in Example 1 were dissolved on the one hand in 25 ml of 10M tris-buffer with pH 8.0 and on the other hand in 25 ml of the same buffer containing 2% of sodium lauryl sulphate (SLS). All solutions were strongly coloured and transferred to dialysis tubes and dialyzed against the solvent (identically the same within and outsides of the tube). The dialysis tube prevents proteins from passing through the tube (to the solution outsides), but admits passage of smaller molecules, e.g. hempeptides. SLS has the ability to stop hydrophobic interaction and thus subdivides larger molecular aggregates to separate molecules. During the dialysis passage of coloured material occurred only in the system wherein the hemiron product was dissolved and dialyzed with tris-buffer containing SLS. In all other cases the colour (the hem) remained inside the tube wall. Special analysis of the coloured material which had passed the tube wall showed that this had the same proportions between iron and amino acids as the material within the tube. This experiment permits the conclusion that hydrophobic interaction causing aggregate formation of hem-containing peptides is the main mechanism (according to alternative a); this is elucidated in Fig. 1F—G.

A continuing enzymatic hydrolysis of hemoglobin according to the invention thus seems to cause such a hydrophobic interaction between hem-containing hemoglobin fragments that these are assembled to aggregates which can be separated from material which does not contain hem instead of, as could be expected, being distributed in the hydrolysis and the rest fractions.

According to the invention it is thus possible by a suitable choice of denaturation method, reaction conditions for the enzymatic hydrolysis of hemprotein and separation method to prepare hemiron products with an increased content of hemiron, by removing the amino acids liberated during the hydrolysis; neither hem nor iron is required to be added from an external source.

A suitable starting material for the present process is whole blood from animals or red blood corpuscles which contain all hemoglobin in blood. The blood corpuscles are the part of the whole blood which remains when the blood plasma has been separated. For use in the present process the red blood corpuscles must be subdivided both when they are used as such or in the form of whole blood. The decomposition is simpliest made by osmotic treatment in the presence of water; the blood corpuscles will burst when water is added and their content of hemoglobin is separated to the solution (hemolysis). The hemolysis is suitably made by adding 1—4 parts of water to whole blood or blood corpuscles.

The denaturation of the hemprotein is suitably made by the addition of alkali, preferably $Ca(OH)_2$ and above all NaOH or KOH, to a pH of 11, whereupon pH 11 is maintained for 30 minutes up to 8 hours; before the hydrolysis the pH is suitably decreased, e.g. with mineral acids, to pH 7—9 depending on the chosen enzyme. Denaturation can also be achieved by the addition of urea or acid or a solvent mixable with water. The hydrolysis can be made by adding a proteolytic enzyme to denatured hemoglobin in a concentration of for instance 1—12% in water, preferably 4—8%. This enzyme can be of an animal origin, i.e. trypsin and chymotrypsin, or a plant enzyme, e.g. papain, bromelain and ficin, or of a microbial origin, suitably an enzyme from microorganisms which are permitted for foodstuff use, e.g. certain bacteria belonging to the genus Bacillus or fungi belonging to the genus Aspergillus. Such microbial enzymes are commercially available, for instance under the trade names Alcalase® and Neutrase® which are obtained from bacillus and have a strength of 6 and 1.5 Anson units/g resp. and they are permitted for use in foodstuffs. All the exemplified enzymes are prepared by a number of different manufacturers and for the present purpose they are equally usable under the condition that they are used in a similar manner. It will be necessary to use amounts of the different enzymes which give the same effect. A way to express the activity of proteolytic enzymes in so-called Anson units (cf. N. L. Davis and E. L. Smith, "Methods of Biochemical Analysis", Vol. II, page 215, Interscience Publishers, New York, 1955). An Anson unit is the amount of enzyme which is required for decomposition of a standard protein to a certain extent during a certain period of time. Commercial enzyme preparations usually contain 0.1—50 Anson units per gram depending on the degree of enrichment.

The enzymatic hydrolysis is suitably performed at a constant temperature, e.g. within the range 10—80°C, suitably 25—60°C, depending on the optimal temperature and stability of the chosen enzyme. The choice of temperature can also be determined on the basis of the desired reaction rate. A change of the temperature to low or high values, e.g. to lesser than 10°C and higher than 80°C, can be used for a heavy decrease of the reaction velocity or a complete stopping of the reaction.

In the hydrolysis pH is another parameter which can be chosen depending on the pH optimum and pH stability of the chosen enzyme, e.g. in the range 4—11. For plant enzymes such as papain pH can be relatively low, e.g. 5, whereas for alkaline microbial proteases a higher value is suitably chosen, e.g. 9—10. The reaction velocity can also be governed with the aid of pH.

In hydrolysis of proteins carboxy groups are liberated which tend to decrease the pH of the reaction mixture. If the pH is allowed to decrease in this manner the pH decrease will continuously change the reaction velocity. If the reaction is started at or below the optimal pH of the enzyme the reaction velocity will gradually decrease, whereas when the starting pH is chosen above the optimal pH of the enzyme the reaction velocity will first increase until the pH optimum is reached, whereupon the velocity will gradually decrease. pH can also be kept constant during the reaction by continuous addition of alkali, suitably NaOH or KOH, preferably in an aqueous solution, the added amount of alkali per time unit also being a measure of the reaction velocity and the total added amount of alkali being a measure of the hydrolysis degree.

The hydrolysis products formed during the reaction will retard the reaction by its blocking action on the enzyme; accordingly, the hydrolysis products also govern the course of the reaction. If these hydrolysis products are allowed to remain in the reaction mixture they will alone or together with other parameters which govern the reaction such as the temperature and the pH influence the velocity of the reaction and the hydrolysis degree. Such conditions prevail in a simple reaction vessel in which the reactants are admixed and the reaction will proceed until it stops.

If the reaction is performed under such conditions that the hydrolysis products are continuously removed the reaction will on the other hand be influenced to a much lesser extent of these products; accordingly, the reaction can be governed to an essential extent as regards velocity and hydrolysis degree in other ways, e.g. with the aid of the temperature and the pH. The hydrolysis products can continuously be removed in several ways, preferably by ultrafiltration. Ultrafiltration is performed by pressing the reaction mixture against a porous membrane which is of such a nature that molecules up to a certain magnitude can pass through the membrane, whereas greater molecules are retained. By choosing a membrane with a suitable pore size it is possible to decide the greatest magnitude of the molecules which pass through the membrane. The properties of the material passing through the membrane as well as the properties of the material which does not pass through the membrane can, like the reaction velocity and the hydrolysis degree, be governed by a suitable choice of reaction and ultrafiltration parameters, such as temperature, pH, protein/enzyme ratio, membrane choice, pressure and circulation conditions, the volume of the reaction mixture, addition of alkali, etc.

Since the hemiron content is the most interesting property of the final preparation in this context the reaction must be performed in such a manner that preferably amino acids and as little as possible of the hemiron is removed. The properties of the hemiron-containing fraction should further be adjusted so that it can be easily separated from the remainder of the material. This is achieved by a suitable

5

# O 061 556

choice of reaction conditions, above all the choice of the enzyme, the hydrolysis pH and the reaction time, precipitation or membrane filtration; the content of hemiron can also be varied in this manner.

In the flow sheet below the invention is elucidated as described above. According to the route in alternative 1 only the hem-containing fraction can be precipitated at pH 4—5. According to alternative 2 only the hem-containing fraction can be concentrated by ultrafiltration. By combination of ultrafiltration and precipitation (alternative 3) also the hem-containing fraction can be concentrated.

The technical properties required in the present hemiron product depend on the intended use. Below it will be shown only how to prepare a hemiron-enriched preparation according to the invention with monitoring of the hemiron enrichment. The product can be adapted to the required technical properties with the aid of the same measures as those used for the hemiron enrichment described above but also other methods can be used which per se do not have any interaction on the hemiron enrichment.

The invention is described in detail in the following examples.

Example 1
Influence of enzyme and pH

To each one of six batches (A—F) each containing 1000 g of dark plasma containing 350 g of protein, 14 g of hem and 1.1 g of iron 1000 g of water were added under stirring for hemolysis of the blood corpuscles. Subsequently each of the batches A—F was admixed with further 3000 g of water and so much 5N aqueous NaOH that the pH was increased to 11.

After 1 hour at room temperature so much 1M aqueous citric acid solution was added to batches A, C and E that the pH decreased to 9.0 and to batches B, D and F a sufficient quantity to make the pH 7.5. All batches were filtrated through paper filters. The temperatures in batches A—F were adjusted to 50°C. Batches A and B were then admixed with 3.5 g of Alcalase®, batches C and D with 14 g Neutrase® and batches E and F with 7 g of papain. All these enzyme preparations, Alcalase®, Neutrase® and papain, were dissolved in 50 mls of 0.1M phosphate buffer of pH 8. The addition of the enzyme preparation started hydrolysis in all batches. During the hydrolysis the changes of pH were monitored with the aid of a pH-meter and 5N aqueous NaOH were added in portions and with such a frequency that pH always was kept within the range 8.7—9 in batches A, C and E and within the range 7.2—7.8 in batches B, D and F. After 2 hours the hydrolysis velocity was decreased by decrease of the temperature to 10°C, whereupon batches A—F were admixed with so much of 3M aqueous HCl that pH in all of the batches decreased to 4.5. In all batches a dark precipitation was formed which was removed by centrifugation and dried; nitrogen and iron analysis was then made on the dried precipitation. Already an ocular inspection of the precipitations and the solutions showed that there were differences as to the hem content. The results of the analysis are given in the following table.

| Batch | Enzyme | pH | Iron content (mg/g) | Degree of enrichment* |
|---|---|---|---|---|
| Dark plasma | | | | |
| A | Alcalase® | 9.0 | 12.9 | 4.0 |
| B | " | 7.5 | 5.2 | 1.6 |
| C | Neutrase® | 9.0 | 5.6 | 1.8 |
| D | " | 7.5 | 6.0 | 1.9 |
| E | Papain | 9.0 | 12.9 | 4.0 |
| F | " | 7.5 | 5.2 | 1.6 |

*Number of times enriched in relation to the iron content of the dark plasma.

This example shows that the enriched iron preparation can be prepared by splitting hemoglobin with the aid of one iron-enriched fraction and one fraction containing amino acids which have been split off and that these fractions are separated by precipitation of the hemiron-enriched fraction. The degree of enrichment at a constant hydrolysis time and temperature has been governed by adjustment of the pH and/or the choice of the enzyme. Alcalase® as well as papain have thus given a considerably greater iron enrichment at pH 9.0 than at pH 7.5, whereas Neutrase® gave essentially the same results at pH 9.0 and 7.5.

6

Example 2

Membrane process

To each one of two batches (A and B), each amounting to 5 kgs of dark plasma containing 1600 g of protein 5 kg of water were added for hemolysis of the blood corpuscles, whereupon denaturation was performed by alkali treatment at pH 11 and filtration analogous with Example 1. Subsequently further 35 kg of water were added so that each batch weighed totally 45 kg, whereupon pH was adjusted to 9.0 with 2M aqueous HCl. After adjustment of the temperature to 50°C in a jacketed steel vessel the both batches were admixed with 22.5 g of Alcalase®, dissolved in 500 ml of 0.1M aqueous phosphate buffer of pH 8.0 for starting the hydrolysis reaction. During the reaction pH was kept in the range 8.8—9.2 in the manner described in Example 1. The hydrolysis was performed at an essentially constant pH by addition of aqueous sodium hydroxide, at first during a short hydrolysis period in the jacketed vessel, this prehydrolysis period being 21 minutes for batch A and 7.5 minutes for batch B. Then the hydrolysis was performed under ultrafiltration which for batch A lasted for 64 minutes and for batch B for 45 minutes. The ultrafiltration in which the iron-enriched fraction was separated from the amino acid fraction was performed in membranes of the type "hollow fibre" with a nominal separation degree of 10,000 dalton for batch A and 7000 dalton for batch B in the manner more closely described in Example 3. The treatment was in both cases stopped when 3.0 moles of NaOH had been added. At that time 5 litres of each batch remained, viz. 11% of the original volume. In both cases the residue was dried and analyzed for iron. The results are given in the following table.

| Batch | Ultrafiltration (Dalton) | Iron (mg/g) | Enrichment degree |
|---|---|---|---|
| Starting material | — | 2.2 | 1 |
| A | 10,000 | 5.2 | 2.4 |
| B | 7,000 | 8.8 | 4.4 |

The enrichment degree for iron can thus be governed with the aid of the porosity of the membrane.

Example 3

Membrane process

Three batches (A—C) each consisting of 5000 g of dark plasma containing about 1750 g hemoglobin, 70 g hem and 5.5 g of iron were hemolyzed and denaturated as described in Example 1.

To batches A and B 1M aqueous citric acid was added for decrease of the pH to 9.0 and to batch C 1M aqueous citric acid was added for decrease of the pH to 7.5. All of the batches were adjusted to 54°C, whereupon enzyme was added. To each one of the batches A and B 18.6 g of Alcalase® were added, dissolved in 400 ml of 0.1M aqueous phosphate buffer filtrated through paper filter and to batch C 74.4 g of Neutrase® in 400 ml of 0.1M aqueous phosphate buffer at pH 7.5 was added, likewise with previous filtration; this started hydrolysis. pH was kept constant between 8.7 and 9.3 as regards the batches A and B and between 7.2 and 7.8 for batch C as described in Example 1. The reaction velocities in batches A, B and C were decreased after 20 minutes, 105 minutes and 210 minutes resp. by decrease of the temperature to about 40°C (for all batches A—C), 2M aqueous HCl also being added to batch B for decrease of the pH to 7.6. Ultrafiltration was then immediately started and the temperature was slowly decreased to the ambient temperature (about 20°C). The ultrafiltration was in all cases performed with a membrane of the type "hollow fibre" with a separation limit (nominal molecular weight exclusion limit) of 50,000 dalton and with an active surface of 2.46 m². The pressure before the membrane was 100 kPa and after the membrane 70 kPa. The flow of the permeate was determined to 90 l/m² · h, viz. the part of the solution which passed through the membrane. When about 5 litres of solution remained water was added in two portions, each consisting of 8 litres, for the purpose of further removing split-off amino acids. Finally, the remaining solution was concentrated by evaporation to about 3.5 litres. The obtained concentrates for batches A—C were dried and weighed, whereupon a nitrogen and iron analysis was performed. The results are given in the following table.

| Sample | Enzyme | Hydrolysis pH | Filtration pH | Time (min) | Iron (mg/g) | Enrichment degree |
|---|---|---|---|---|---|---|
| Dark plasma | — | — | — | — | 3.3 | 1.0 |
| A | Alcalase® | 9.0 | 9.0 | 20 | 10.4 | 3.2 |
| B | Alcalase® | 9.0 | 7.6 | 105 | 18.8 | 5.7 |
| C | Neutrase® | 7.5 | 7.5 | 210 | 12.2 | 3.7 |

7

# 0 061 556

The results show that an enriched hemiron preparation can be prepared by hydrolyzation of hemoglobin with an enzyme to the formation of an iron-enriched fraction and a second fraction containing split-off amino acids and by separating these fractions by ultrafiltration with the use of a membrane with a separating degree of 50,000 dalton and that a lower filtration pH is favourable for the enrichment degree.

The yield of hemiron-enriched fraction was shown to be greater when the pH at the ultrafiltration was 7 instead of 9. By this adjustment of the pH before the ultrafiltration it is possible to use a more porous membrane than could be expected on the basis of the molecular weight ratios for hemoglobin and hemiron-enriched (hem-enriched) fraction. In this manner the ultrafiltration will be more rapid. It can also be seen that the hemiron enrichment can be governed by the choice of hydrolysis time, the same enzyme and the same pH being used, or by the choice of enzyme.

Example 4

Pretreatment and hydrolysis of dark plasma were performed analogous with Example 3, the pH during the hydrolysis being kept at 9. Ultrafiltration was performed in a first case (A) with a membrane with the separation degree of 10,000 dalton and in a second case (B) first through a membrane with the separation degree of 50,000 dalton followed of filtration through a membrane with the separation degree of 10,000 dalton. In case B relatively more hemiron passed the membrane with the separation degree of 50,000 dalton, since the pH at the ultrafiltration was 9, as compared with Example 3, wherein pH was 7. The hemiron fraction from the filtrate in case B could be concentrated by ultrafiltration of the first filtrate once more but through a membrane with the separation degree of 10,000 dalton. In both cases (A, B) the material was recovered when 5 litres remained, whereupon pH in the both cases was decreased to 4.5 by the addition of hydrochloric acid. Precipitations were obtained which were recovered, dried, weighed and analyzed as to the iron content.

The results are shown in the following table.

| Sample | Iron (mg/g) | Enrichment degree |
|---|---|---|
| Dark plasma | 1.8 | 1.0 |
| Precipitation A | 19.5 | 6.9 |
| Precipitation B | 46.1 | 16.5 |

This example shows that the enrichment degree can be governed by different combinations of ultrafiltration and precipitation. The materials (precipitations A and B) prepared according to this example thus consist of 1.95 and 4.6% of iron resp. which corresponds to 22 and 54% hem (iron×11.64=hem); thus they contain 78 and 46% resp. of other materials which mainly consist of amino acids.

The examples show that hemiron preparations can be prepared by enzymatic hydrolysis of hemoglobin to the formation of one hemiron-enriched amino acid fraction and one amino acid fraction without hemiron and that these two fractions can be separated, e.g. by precipitation of the hemiron-enriched fraction or by ultrafiltration or by a combination of these methods. The hemiron-enriched fraction still contains amino acids which play an important role for the transport and resorption of the hemiron. The yield of iron, calculated on the iron content of the original dark plasma, was about 80% in all of the examples.

The most suitable specific form for the preparation of the present hemiron-enriched amino acid preparation depends on its use. For achieving the best hemiron enrichment the specific form according to Example 1B is most suitable when working without membrane technique, with the use of membrane technique the specific form according to Example 3B is most suitable and the combination of membrane technique and precipitation is most suitable according to the specific form in Example 4B.

8

**0 061 556**

Flow sheet for alternative processes for the preparation
of hemiron-enriched preparations.

Red blood corpuscles — (remainder after separation of plasma)

↓

Hemolysis — (dilution with water)

↓

Denaturation — (alkali, urea, acid, heat)

↓

Hydrolysis — (proteolytic enzyme, pH, temperature, time)

↓

Separation

↓

Alternative 1                                          Alternative 2

Precipitation (pH)                                     Ultrafiltration

Amino acid fraction    Hemiron-enriched product    Hemiron-enriched product    Amino acid fraction

Alternative 3 +precipitation

↓

Highly enriched hemiron product

## Claims

1. Hemiron-enriched amino acid preparation prepared from hemproteins, characterized in that the iron content of the preparation solely consists of the porphyrin iron of the original hemprotein bonded in this form (hem) to amino acid chains which are considerably shorter than the amino acid chains in the original hemprotein, but which forms aggregates which have larger dimensions than the separate, short, hemiron-containing amino acid chains.

2. Amino acid preparation according to claim 1, characterized by a constant ratio between hem and iron and by a porphyrin hemiron content of at least 0.5% and preferably at the most 5%, calculated as the iron which is bonded to porphyrin.

3. A process for the preparation of hemiron-enriched amino acid preparations from hemproteins, especially hemoglobin, characterized in that the hemprotein in a manner known per se is first hemolyzed and denaturated and subsequently split up with the aid of proteolytic enzymes to the formation of one hemiron-enriched and one fraction which does not contain hemiron and that the hemiron-enriched fraction is recovered by separation and is stabilized by heat treatment and dewatering, preferably drying.

4. A process according to claim 3, characterized in that the enzyme used is preparations containing proteolytic enzymes from animals, plants, microorganisms or fungi.

5. A process according to claim 3 or 4, characterized in that the hydrolysis is performed in the pH range 4—11.

6. A process according to any one of claims 3—5, characterized in that the hydrolysis is performed at a temperature of 10—80°C.

7. A process according to any one of claims 3—6, characterized in that the hemiron-enriched

9

## 0 061 556

fraction is separated from the amino acid-enriched fraction by precipitation at a pH of 4—5 or by membrane techniques such as ultrafiltration.

8. A process according to claim 7, characterized in that the ultrafiltration is performed at a pH of 5—9.

9. A process according to claim 7 or 8, characterized in that the separation is performed with the use of a membrane which has a separation degree of 1000—50,000 dalton or with a combination of membranes with a separation degree of 1000—50,000 dalton.

10. A process according to any one of claims 3—9, characterized in that the hemiron-enriched fraction is separated from the amino acid-enriched fraction by a combination of ultrafiltration and precipitation.

**Patentansprüche**

1. Hemiron-angereichertes Aminosäurepräparat, hergestellt aus Hemproteinen, dadurch gekennzeichnet, daß der Eisengehalt des Präparates ausschließlich aus dem Porphyrin-Eisen des ursprünglichen Hemproteins besteht, das in dieser Form (hem) zu Aminosäureketten gebunden ist, welche beachtlich kürzer sind, als die Aminosäureketten im ursprünglichen Hemprotein, welches jedoch Aggregate bildet, die größere Ausmaße haben, als die separaten, kurzen Hemiron beinhaltenden Aminosäureketten.

2. Aminosäurepräparat nach Anspruch 1, gekennzeichnet durch ein konstantes Verhältnis zwischen Hem und Eisen, und durch einen Porphyrin Hemiron Anteil von mindestens 0,5% und vorzugsweise maximal 5%, gerechnet als das Eisen, das mit dem Porphyrin gebunden ist.

3. Ein Verfahren zur Herstellung von Hemiron angereicherten Aminosäurepräparaten aus Hemproteinen, insbesondere Hämoglobin, dadurch gekennzeichnet, daß das Hemprotein in an sich bekannter Weise zuerst homolysiert und denaturiert, und anschließend mit der Hilfe proteolytischer Enzyme gespalten wird, zur Bildung eines Hemiron angereicherten Teiles und eines Teiles, der Hemiron nicht enhält, und daß der Hemiron-angereicherte Teil durch Trennung wiedergewonnen wird, und durch Hitzebehandlung und Entwässerung, vorzugsweise Trocknung, stabilisiert wird.

4. Ein Verfahren nach Anspruch 3, dadurch gekennzeichnet, daß das verwendete Enzym Präparate sind, die proteolytische Enzyme von Tieren, Pflanzen, Mikroorganismen oder Pilzen enthalten.

5. Ein Verfahren nach Anspruch 3 oder 4, dadurch gekennzeichnet, daß die Hydrolyse im pH-Bereich 4—11 durchgeführt wird.

6. Ein Verfahren nach einem der Ansprüche 3 bis 5, dadurch gekennzeichnet, daß die Hydrolyse bei einer Temperatur von 10 bis 80°C durchgeführt wird.

7. Ein Verfahren nach einem der Ansprüche 3 bis 6, dadurch gekennzeichnet, daß der Hemiron-angereicherte Teil vom Aminosäure angereicherten Teil durch Fällung bei einem pH von 4—5 oder durch Membrantechniken wie Ultrafiltration getrennt wird.

8. Ein Verfahren nach Anspruch 7, dadurch gekennzeichnet, daß die Ultrafiltration bei einem pH von 5—9 durchgeführt wird.

9. Ein Verfahren nach Anspruch 7 oder 8, dadurch gekennzeichnet, daß die Trennung mit einer Membran durchgeführt wird, die einen Trennungsgrad von 1000—50.000 Dalton hat, oder mit einer Kombination von Membranen mit einem Trennungsgrad von 1000—50.000 Dalton.

10. Ein Verfahren nach einem der Ansprüche 3 bis 9, dadurch gekennzeichnet, daß der Hemiron-angereicherte Teil vom Aminosäureangereicherten Teil durch eine Kombination von Ultrafiltration und Fällung getrennt wird.

**Revendications**

1. Composition d'acides aminés enrichie en fer hématinique, préparée à partir d'hématoprotéines, caractérisée en ce que le contenu en fer la composition ne consiste qu'en fer porphyrinique de l'hématoprotéine originelle lié sous cette forme (hématinique) aux chaînes d'acides aminés qui sont notablement plus courtes que les chaînes d'acides aminés de l'hématoprotéine originelle, mais qui forme des agrégats de dimensions plus importantes que les chaînes d'acides aminés courtes et séparées contenant le fer hématinique.

2. Composition d'acides aminés selon la revendication 1, caractérisée par un rapport constant entre l'hématine et le fer et par une teneur en fer hématinique de la porphyrine d'au moins 0,5% et, de préférence d'au moins 5%, calculée en fer lié à la porphyrine.

3. Procédé de préparation de compositions d'acides aminés enrichies en fer hématinique à partir d'hématoprotéines et, en particulier, d'hémoglobine, caractérisé en ce que l'hématoprotéine est, d'une manière connue en soi, tout d'abord hémolysée, puis dénaturée et ensuite séparée à l'aide d'enzymes protéolytiques pour former une partie enrichie en fer hématinique et une partie exempte de fer hématinique, et en ce que la partie enrichie en fer hématinique est récupérée par séparation et stabilisée par traitement à la chaleur et élimination d'eau, de préférence par séchage.

4. Procédé selon la revendication 3, caractérisé par le fait que l'enzyme utilisé est constitué par

10

des compositions contenant des enzymes protéolytiques provenant d'animaux, plantes, micro-organismes ou champignons.

5. Procédé selon l'une des revendications 3 ou 4, caractérisé en ce que l'hydrolyse est effectuée dans une gamme de pH de 4 à 11.

6. Procédé selon l'une des revendications 3 à 5, caractérisé en ce que l'hydrolyse est effectuée à une température de 10 à 80°C.

7. Procédé selon l'une des revendications 3 à 6, caractérisé en ce que la partie enrichie en fer hématinique est séparée de la partie enrichie en acides aminés par précipitation à un pH de 4 à 5 ou par des techniques à membranes telles que l'ultrafiltration.

8. Procédé selon la revendication 7, caractérisé en ce que l'ultrafiltration est effectuée à un pH de 5 à 9.

9. Procédé selon l'une des revendications 7 ou 8, caractérisé en ce que la séparation est effectuée à l'aide d'une membrane ayant un degré de coupure de 1.000 à 50.000 daltons ou d'une combinaison de membranes avec un degré de coupure de 1.000 à 50.000 daltons.

10. Procédé selon l'une des revendications 3 à 9, caractérisé en ce que la partie enrichie en fer hématinique est séparée de la partie enrichie en acides aminés par une combinaison d'ultrafiltration et de précipitation.

FIGURE 1.    PRINCIPLES FOR PREPARING HEMIRON-ENRICHED PRODUCTS

A

Denaturation
(alkali)

B

Four wound-up chains
Iron content = 0.35%

▣ = hemgroup
     (porfyrin with central iron atom)

Four straightened-out chains

C

Hydrolysis
(enzyme)

D

E

Each chain is decomposed
by a proteolytic enzyme
(arrows)

Low molecular amino acid
fraction cannot be
precipitated, will pass
membrane;
iron content <0.35%

Hemgroup on a short
amino acid chain,
low molecular,
iron content >0.35%

F

Aggregation

G

Several small amino acid chain parts with
hemgroup combine to aggregates insoluble at
pH 4.5 and cannot pass the membrane

0 061 556